(19) Europäisches Patentamt
European Patent Office
Office européen des brevets

(11) Veröffentlichungsnummer : **0 003 500 B2**

# (12) NEUE EUROPÄISCHE PATENTSCHRIFT

(45) Veröffentlichungstag der neuen Patentschrift :
**02.01.92 Patentblatt 92/01**

(51) Int. Cl.$^5$ : **C07D 249/08**

(21) Anmeldenummer : **79100059.9**

(22) Anmeldetag : **10.01.79**

## (54) Verfahren zur Herstellung von 1,2,4-Triazol.

Die Akte enthält technische Angaben, die nach dem Eingang der Anmeldung eingereicht wurden und die nicht in dieser Patentschrift enthalten sind.

(30) Priorität : **20.01.78 DE 2802491**

(43) Veröffentlichungstag der Anmeldung :
**22.08.79 Patentblatt 79/17**

(45) Bekanntmachung des Hinweises auf die Patenterteilung :
**28.10.81 Patentblatt 81/43**

(45) Bekanntmachung des Hinweises auf die Entscheidung über den Einspruch :
**02.01.92 Patentblatt 92/01**

(84) Benannte Vertragsstaaten :
**BE CH DE FR GB IT NL**

(56) Entgegenhaltungen :
**LU-B- 61 617**
**JOURNAL OF THE AMERICAN CHEM. SOC., Vo. 77, 10-02-1955 C. AINSWORTH et al.; "Isomeric and nuclear-substituted beta-aminoethyl-1,2,4-triazoles", Seiten 621-624**

(73) Patentinhaber : **BAYER AG**
**W-5090 Leverkusen 1 Bayerwerk (DE)**

(72) Erfinder : **Kaiser, Reinhard, Dr.**
**Morgengraben 14**
**W-5000 Köln 80 (DE)**
Erfinder : **Steffan, Guido, Dr.**
**Herzogenfeld 52**
**W-5068 Odenthal (DE)**

EP 0 003 500 B2

**Beschreibung**

Die Erfindung betrifft ein Verfahren zur Herstellung von 1,2,4-Triazol durch Umsetzung von Hydrazin mit Formamid.

Es ist bekannt (LU 61 617), 1,2,4-Triazol durch Umsetzung von Hydrazin mit Formamid im Temperaturbereich von 90 bis 260 ° mit einem Molverhältnis von 1 zu 3 herzustellen.

Weiterhin ist aus J.Amer.Chem.Soc 77, Seite 621 (1955) bekannt, dass bei dem Erhitzen von Hydrazin und Formamid im Molverhältnis 1:2 bei Atmosphärendruck 1,2,4-Triazol mit maximal 30% der Theorie Ausbeute, bezogen auf das Hydrazin, entsteht. Als Hauptprodukt entsteht bei der Umsetzung N,N'-Diformylhydrazin.

Aus der gleichen Literaturstelle ist ebenfalls bekannt, dass 1,2,4-Triazol beim Erhitzen von N,N'-Diformylhydrazin mit einem Überschuss von Ammoniak (ca. 18 Mol Ammoniak pro Mol N,N'-Diformylhydrazin) unter Druck (Autoklaven) mit einer Ausbeute von 70 bis 80% der Theorie, bezogen auf N,N'-Diformylhydrazin (entspricht 56 bis 54% der Theorie, bezogen auf das Hydrazin) entsteht.

Es wurde nur ein Verfahren zur Herstellung von 1,2,4-Triazol durch Umsetzung von Hydrazin mit Formamid gefunden, das dadurch gekennzeichnet ist, dass man im Temperaturbereich von 100 bis 250 °C in Gegenwart von Ammoniak Hydrazin mit Formamid umsetzt, wobei man Hydrazin, Formamid und Ammoniak im Molverhältnis von 1:2,0:1 bis 1:2,7:0,3 einsetzt.

Das erfindungsgemässe Verfahren kann durch die folgende Reaktionsgleichung erläutert werden:

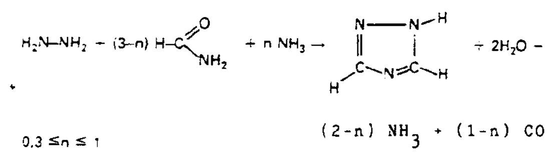

Als Ammoniak für das erfindungsgemässe Verfahren kann handelsübliches Ammoniak eingesetzt werden, das im allgemeinen gasförmig oder gelöst in einer Mischung der Ausgangs- und/oder der Reaktionsprodukte in das Reaktionsgemisch geleitet wird.

Das erfindungsgemässe Verfahren wird in Gegenwart des bei der Umsetzung entweichenden Ammoniaks durchgeführt, das ganz oder teilweise in die Umsetzung zurückgeführt wird.

Die Konzentration des Ammoniaks in der Umsetzung kann in weiten Grenzen schwanken; es ist jedoch zweckmässig, jeweils soviel Ammoniak einzuleiten, dass das Reaktionsgemisch praktisch mit Ammoniak gesättigt ist.

Hydrazin tür das erfindungsgemäss Verfahren kann sowohl wasserfrei als auch als Hydrazinhydrat eingesetzt werden. Vorzugsweise setzt man Hydrazinhydrat ein, das in konzentrierter oder in verdünnter wässriger Lösung vorliegen kann. Im allgemeinen verwendet man eine wässrige Hydrazinlösung mit einem Gehalt von 20 bis 70 Gew.-%, bevorzugt von 60 bis 70 Gew.-%, Hydrazin.

Das Formamid tür das erfindungsgemässe Verfahren kann in handelsüblicher Reinheit eingesetzt werden.

Nach dem erfindungsgemässen Verfahren werden Hydrazin, Formamid und Ammoniak im Molverhältnis von 1:2,0:1 bis 1: < 2,7: > 0,3, bevorzugt von 1:2,4:0,9 bis 1:2,6:0,4, eingesetzt.

Das erfindungsgemässe Verfahren wird im Temperaturbereich von 100 bis 250 °C, bevorzugt von 110 bis 220 °C durchgeführt.

Das erfindungsgemässe Verfahren kann bei Normaldruck, erhöhtem oder vermirdertem Druck durchgeführt werden. Im allgemeinen ist es zweckmässig, das erfindunsgemässe Verfahren unter Normaldruck auszuführen; die Anwendung von geringem Unterdruck hat jedoch besonders in der letzten Phase der Umsetzung den Vorteil, dass Wasserdampt und gasförmige Abfallprodukte leichter entfernt werden.

Es ist selbstverständlich auch möglich, das erfindungsgemässe Verfahren in Gegenwart eines Inertgases, z.B. Stickstoff, durchzuführen.

Das erfirdungsgemässe Verfahren kann sowohl diskontinuierlich als auch kontinuierlich durchgeführt werden.

In einer bevorzugten Ausführungsform des erfindungsgemässen Verfahrens führt man die Umsetzung kontinuierlich in einer mehrstufigen Reaktorkaskade durch. Insbesondere bevorzugt ist die Verwendung von 3- und 4-stutigen Reaktorkaskaden.

Besonders vorteilhaft lässt sich das erfindungsgemässe Verfahren durchführen, wenn man kontinuierlich Hydrazinhydrat mit Formamid in einer ersten Stufe der Reaktorkaskade im Molverhältnis von 1 bis 2,4 bis 1 zu 2,7 bei Temperaturen von 100 bis 120 °C umsetzt, das Reaktionsgemisch anschliessend in einer zweiten

Stufe bei 180 bis 200 °C zur Reaktion bringt, die dabei entstehenden gasförmigen ammoniakhaltigen Reaktionsprodukte auf 110 bis 150 °C abkühlt und das anfallende Kondensat in die zweite Stufe zurückführt und das in der zweiten Stute anfallende gesamte Reaktionsgemisch anschliessend in einer dritten Stufe bei 210 bis 230 °C umsetzt, die dabei entstehenden gasförmigen ammoniakhaltigen Reaktionsprodukte auf 40 bis 80 °C abkühlt und das Kondensat in die erste Stufe zurückführt und das Reaktionsprodukt der dritten Stufe kontinuierlich entnimmt.

Bei dieser Verfahrensweise werden die gasförmigen Reaktionsprodukte, die im wesentlichen Ammoniak enthalten, ganz oder teilweise in die jeweiligen Reaktionsstufe zurückgeführt.

Das erfindungsgemässe Verfahren lässt sich aber auch so gestalten, dass man das in der ersten Reaktionsstufe ammoniakhaltige gasförmige Reaktionsprodukt in die zweite Reaktionsstufe führt und den mit den flüchtigen Reaktionsprodukten aus der zweiten Stufe entweichenden Ammoniak zumindestens teilweise mit dem Kondensat in die zweite Stufe zurückführt, die aus der zweiten Reaktionsstufe mit dem Reaktionsgemisch in die dritte Stufe geführten und mit dem flüchtigen Reaktionsprodukt aus dieser dritten Stufe entweichenden Ammoniak ganz oder teilweise in die erste Stufe zurückleitet.

In einer weiteren Ausführungsform wird das in der ersten und zweiten Stufe entstehende Reaktionsgemisch vollständig in eine dritte Stufe geleitet. In der dritten Stufe werden die flüchtigen Reaktionsprodukte auf Temperaturen von 60 bis 120 °C abgekühlt und das Kondensat ganz oder teilweise das entweichende Ammoniak in die erste Stufe zurückgeleitet.

Besonders vorteilhaft lässt sich das erfindungsgemässe Verfahren zur Herstellung von 1,2,4-Triazol durchführen, wenn man die Reaktion in einer vierstufigen Reaktorkaskade ablaufen lässt, die Temperatur in den ersten drei Kaskadenstufen wie bei der dreistufigen Reaktorkaskade wählt und in der vierten Stufe die Temperatur auf 195 bis 240 °C erhöht. In diesem Fall werden zweckmässigerweise die in der zweiten Stufe entstehenden flüchtigen Reaktionsprodukte auf 110 bis 140 °C abgekühlt und das anfallende Kondensat in die zweite Stufe zurückgeführt. Die in der dritten und vierten Stufe anfallenden flüchtigen Reaktionsprodukte werden gemeinsam auf 40 bis 80 °C abgekühlt und das Kondensat in die erste Stufe zurückgeführt. Nach der vierten Stufe wird das Endprodukt kontinuierlich abgezogen und isoliert. Selbstverständlich können die aus der dritten und vierten Reaktionsstufe entweichenden flüchtigen Reaktionsprodukte auch getrennt abgekühlt und die Kondensate getrennt oder gemeinsam zurückgeführt werden.

Die einzelnen Verfahrensvarianten lassen sich bei unterschiedlichen mittleren Verweilzeiten in den einzelnen Reaktionsstufen der Reaktorkaskade durchführen. Unter mittlerer Verweilzeit versteht man den Quotienten aus Füllvolumen des Reaktors und der Summe der Volumina der Ausgangsprodukte bei 20 °C, die pro Stunde in den ersten Reaktor eingebracht werden.

Die Verweilzeit in der ersten Reaktorstufe beträgt im allgemeinen 5 bis 150 Minuten, vorzugsweise 20 bis 40 Minuten. Die Verweilzeiten in den folgenden Reaktorstufen betragen etwa 20 bis 200 Minuten, bevorzugt 40 bis 120 Minuten.

Das nach der letzten Stufe erhaltene Reaktionsgemisch lässt man abkühlen und erhält in hohen Ausbeuten 1,2,4-Triazol, das ohne weitere Reinigung in weitere Umsetzungen eingesetzt werden kann.

1,2,4-Triazol ist ein Zwischenprodukt für die Synthese von Farbstoffen, optischen Aufhellern, Alterungsschutzmittel, pharmazeutischen Präparaten und Schädlingsbekämpfungsmittel (BE 715 569 und BE 729 878 und US 3 293 259).

Beispiel 1

In einen 1 l Mehrhalskolben werden gleichzeitig 655 g/h Formamid und 280 g/h Hydrazinhydrat kontinuierlich bei 110-115 °C eindosiert. Als unter den Reaktionsbedingungen gasförmige Reaktionsprodukte entweichen Wasser und Ammoniak.

Die im 1. Reaktionskolben unter den Reaktionsbedingungen flüssig vorliegende Reaktionsmischung läuft kontinuierlich über einen Überlauf in einen 2 l Mehrhalskolben der auf ca. 190 °C geheizt wird. Die gasförmig entweichenden Produkte werden auf ca. 140 °C abgekühlt und das dabei anfallende Kondensat in das 2. Reaktionsgefäss zurückgeleitet.

Die im 2. Kolben unter den Reaktionsbedingungen flüssig vorliegenden Reaktionsmischung läuft kontinuierlich in einen 1,5 l Mehrhalskolben, der auf ca. 215-220 °C gehalten wird. Die gasförmig entweichenden Produkte werden auf 50-55 °C abgekühlt und das dabei anfallende Kondensat in den 1. Kolben zurückgeleitet.

Die den 3. Reaktionskolben verlassende unter den Reaktionsbedingungen flüssig vorliegende Reaktionsmischung wird abgekühlt und erstarrt dabei.

Ausbeute: 382 g/h 95%iges 1,2,4-Triazol
94% der Theorie, bezogen auf Hydrazinhydrat

Fp.: 112-116 °C.

Beispiel 2

Die Durchführung erfolgt wie in Beispiel 1, die im 3. Reaktionskolben unter den Reaktionsbedingungen flüssig vorliegende Reaktionsmischung läuft jedoch kontinuierlich in einen 1,5 l Mehrhalskolben, der auf 215-220 °C gehalten wird. Die gasförmig entweichenden Produkte werden auf 50-55 °C abgekühlt und in den 1. Kolben zurückgeleitet. Das 1,2,4-Triazol wird dann wie im Beispiel 1 beschrieben isoliert.

Ausbeute: 367 g/h 97,8%iges 1,2,4-Triazol
93% der Theorie, bezogen auf
Hydrazinhydrat

Fp.: 114-116 °C.

Beispiel 3

In einen 1 l Mehrhalskolben werden gleichzeitig 486 g/h Formamid und 270 g/h Hydrazinhydrat kontinuierlich bei 110-115 °C eindosiert. Die im 1. Reaktionsgefäss unter den Reaktionsbedingungen flüssig vorliegende Reaktionsmischung läuft kontinuierlich über einen Überlauf in einen 2 l Mehrhalskolben der bei 160 °C gehalten wird. Die unter den Reaktionsbedingungen im 1. Reaktor entstehenden gasförmigen Reaktionsprodukte werden mehrere Male durch das im Reaktor 2 vorliegende Reaktionsgemisch geleitet. Die Reaktionsmischung des Reaktors 2 läuft kontinuierlich in einen 1 l Mehrhalskolben über, welcher bei 160°C gehalten wird.

Die Reaktor 2 verlassenden gasförmigen Produkte werden mehrere Male durch das im Reaktor 3 vorliegende Reaktionsgemisch beleitet.

Die Reaktor 3 verlassenden gasförmigen Produkte werden auf 50-80 °C abgekühlt. Das Kondensat wird in Reaktor 1 zurückgeleitet.

Die Reaktionsmischung aus Reaktor 3 läuft über einen Überlauf in einen Glaskolben und wird dort auf 20-30 °C abgekühlt.

Man erhält 389 g/h 90%iges 1,2,4-Traizol
(= 94% der Theorie, bezogen auf
Hydrazinhydrat).

Beispiel 4

Die Reaktion wird wie in Beispiel 3 durchgeführt. In diesem Fall werden jedoch die aus dem 1. Reaktor entweichenden gasförmigen Reaktionsprodukte nicht durch das im 2. Reaktor vorliegende Reaktionsgemisch hindurchgeleitet, sondern gemeinsam mit den aus Reaktor 2 entweichenden gasförmigen Reaktionsprodukten mehrmals durch das im 3. Reaktor vorliegende flüssige Reaktionsgemisch geleitet.

Man erhält 381 g/h ca. 91%iges 1,2,4-Triazol
(= 93% der Theorie, bezogen auf
Hydrazinhydrat).

## Patentansprüche

1. Verfahren zur Herstellunb von 1,2,4-Triazol durch Umsetzung von Hydrazin mit Formamid in einer mehrstufigen Reaktionskaskade unter Normaldruck oder geringem Unterdruck, dadurch gekennzeichnet, daß man die Umsetzung von Hydrazin mit Formamid im Molverhältnis von 1 : 2 bis 1 : 2,7 so durchführt, daß man kontinuierlich Hydrazin mit Formamid in einer ersten Stufe einer Reaktorkaskade bei einer Temperatur von 100 - 120°C umsetzt, das Reaktionsgemisch anschließend in einer zweiten Stufe bei 160 - 200°C zur Reaktion bringt, die dabei entstehenden flüchtigen Reaktionsprodukte auf 110 - 150°C abkühlt und das anfallende Kondensat in die zweite Stufe zurückführt und das in der zweiten Stufe anfallende gesamte Reaktionsgemisch anschließend in einer dritten Stufe bei 160 - 230°C umsetzt, die dabei entstehenden flüchtigen Reaktionsprodukte auf 40 - 80°C abkühlt, das Kondensat in die erste Stufe zurückführt und aus der dritten Stufe das Endprodukt kontinuierlich entnimmt.

2. Verfahren nach Anspruch 1, dadurch gekennzeichnet, daß man dem Verfahren vor der kontinuierlichen Entnahme des Reaktionsproduktes eine vierte Reaktionsstufe anschließt, wobei die Temperaturen in den

ersten drei Kaskadenstufen wie bei der dreistufigen Reaktorkaskade gewählt werden und man in der vierten Stufe die Temperatur auf 195 - 240°C erhöht und wobei man die in der zweiten Stufe entstehenden flüchtigen Reaktionsprodukte auf 110 - 140°C abkühlt und das anfallende Kondensat in die zweite Stufe zwurückführt und die in der dritten und vierten Stüfe anfallenden flüchtigen Reaktionsprodukte getrennt oder gemeinsam auf 40 - 80°C abkühlt und das oder die Kondensat(e) getrennt öder gemeinsam in die erste Stufe zurückführt.

## Claims

1. Process for the preparation of 1,2,4-triazole by reacting hydrazine with formamide in a multi-stage cascade of reactors under atmospheric pressure or slight subatmospheric pressure, characterised in that the reaction of hydrazine with formamide is carried out in the molar ratio of 1:2 to 1:2.7 in such a manner that hydrazine is continuously reacted with formamide in the first stage of a cascade of reactors at a temperature of 100 - 120°C, the reaction mixture is subsequently reacted in a second stage at 160 - 200°C, the resulting volatile reaction products are cooled to 110 - 150°C, and the condensate obtained is recycled into the second stage, and the entire reaction mixture obtained in the second stage is subsequently reacted in a third stage at 160 - 230°C, the resulting volatile reaction products are cooled to 40 - 80°C, the condensate is recycled into the first stage, and the end product is continuously withdrawn from the third stage.

2. Process according to Claim 1, characterised in that a fourth reaction stage is added to the process before the reaction product is withdrawn continuously, the temperatures in the first three stages of the cascade being selected as in the three-stage cascade of reactors, and the temperature being increased in the fourth stage to 195 - 240°C, the volatile reaction products resulting in the second stage being cooled to 110 - 140°C, and the condensate obtained being recycled into the second stage, and the volatile reaction products obtained in the third and fourth stages being cooled to 40 - 80°C, either separately or jointly, and the condensate, or the condensates, being recycled into the first stage, either separately or jointly.

## Revendications

1. Procédé de préparation de 1,2,4-triazole par réaction d'hydrazine avec du formamide dans une cascade de réacteurs à plusieurs étages sous pression normale ou faiblement réduite, caractérisé en ce qu'on effectue la réaction de l'hydrazine avec la formamide dans un rapport molaire de 1:2 à 1:2,7 de façon telle qu'on fait réagir continuellement l'hydrazine avec le formamide dans un premier étage d'une cascade de réacteurs à une température de 100 à 120°C, puis on fait réagir le mélange réactionnel à une température de 160 à 200°C dans un deuxième étage, on refroidit, à 110-150°C, les produits réactionnels volatils ainsi formés, on recycle le condensat obtenu au deuxième étage, puis, dans un troisième étage, on fait réagir, à une température de 160 à 230°C, tout le mélange réactionnel formé dans le deuxième étage, on refroidit, à 40-80°C, les produits réactionnels volatils ainsi formés, on recycle le condensat au premier étage et on retire continuellement le produit final du troisième étage.

2. Procédé suivant la revendication 1, caractérisé en ce qu'on ajoute au procédé, avant l'enlèvement continu du produit de réaction, un quatrième étage de réaction en choisissant pour les trois premiers étages de la cascade, la même température que dans la cascade de réacteurs à trois étages et on porte la température à 195 - 240°C dans le quatrième étage, on refroidit à 110-140°C les produits réactionnels volatils se formant dans le deuxième étage, on recycle le condensat formé au deuxième étage, on refroidit à 40 - 80°C ensemble ou séparément les produits réactionnels volatils se formant dans le troisième et le quatrième étage et on recycle le ou les condensat(s) séparément ou ensemble au premier étage.